(19) Europäisches Patentamt
European Patent Office
Office européen des brevets

(11) Veröffentlichungsnummer : **0 089 574**
**B1**

(12) # EUROPÄISCHE PATENTSCHRIFT

(45) Veröffentlichungstag der Patentschrift : **02.01.86**

(21) Anmeldenummer : **83102401.3**

(22) Anmeldetag : **11.03.83**

(51) Int. Cl.$^4$ : **C 01 B 33/28**, B 01 J 29/36

(54) **Niob-haltige Alumino- und Borosilikatzeolithe, deren Herstellung und Verwendung als Katalysator.**

(30) Priorität : **19.03.82 DE 3210062**

(43) Veröffentlichungstag der Anmeldung : **28.09.83 Patentblatt 83/39**

(45) Bekanntmachung des Hinweises auf die Patenterteilung : **02.01.86 Patentblatt 86/01**

(84) Benannte Vertragsstaaten : **BE DE FR GB NL**

(56) *Entgegenhaltungen :*
**EP-A- 0 042 225**
**EP-A- 0 051 741**
**DE-A- 2 017 807**
**DE-A- 2 746 790**
**DE-A- 2 831 631**
**US-A- 3 702 886**

(73) Patentinhaber : **BASF Aktiengesellschaft**
**Carl-Bosch-Strasse 38**
**D-6700 Ludwigshafen (DE)**

(72) Erfinder : **Hoelderich, Wolfgang, Dr.**
**Mannheimer Strasse 18 C**
**D-6710 Frankenthal (DE)**
Erfinder : **Mross, Wolf Dieter, Dr.**
**Anselm-Feuerbach-Strasse 21**
**D-6710 Frankenthal (DE)**
Erfinder : **Schwarzmann, Matthias, Dr.**
**Carl-Bosch-Strasse 54**
**D-6703 Limburgerhof (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft neue Alumino- und Borosilikatzeolithe, die Niob enthalten, und ein Verfahren zur Herstellung dieser kristallinen Zeolithe in etherischem, alkoholischem oder aminhaltigem Medium sowie die Verwendung dieser Zeolithe als Katalysatoren für die Umwandlung von Methanol und/oder Dimethylether zu niederen Olefinen.

Bor-Zeolithe sind bisher in der Natur nicht gefunden worden. Man erhält sie auf synthetischem Wege z. B. nach einem in DE-OS-2 746 790 beschriebenen Verfahren. Al-haltige zeolithische Materialien sind sowohl natürlicher als auch synthetischer Herkunft. Boro- und Alumino-silikatzeolithe haben sich als katalytisch wirksam erwiesen für verschiedene Kohlenwasserstoffumwandlungen. Die Aluminium-Zeolithe finden in der Technik z. B. zum Cracken von Kohlenwasserstoffen, als Ionenaustauscher und Molekularsiebe Verwendung.

Allgemein besitzen Zeolithe eine hochgeordnete Struktur mit einem starren dreidimensionalen Netzwerk von $SiO_4$- und $MeO_4$-Tetraedern, wobei Me ein dreiwertiges Metall, z. B. Al, Fe, Ga und B sein kann. Diese Tetraeder sind durch gemeinsame Sauerstoffatome verbunden. Das Verhältnis der Si- und Me-Atome zu Sauerstoff beträgt 1 : 2. Die Elektrovalenz der Atome dreiwertiger Elemente enthaltenden Tetraeder ist durch Einschluß von Kationen in den Kristall z. B. eines Alkali- oder Wasserstoffions ausgeglichen. Ein Kationenaustausch ist möglich. Die Räume zwischen den Tetraedern sind vor der Dehydratation durch Trocknen bzw. Calcinieren von Wassermolekeln besetzt.

In den letzten Jahren gewannen kristalline Bor- und Aluminosilikate mit einem hohen $SiO_2/Al_2O_3$-Verhältnis 11 zunehmendes Interesse. Derartige Zeolithe besitzen Pentasilstruktur und zeichnen sich durch hohe thermische Stabilität und außerordentlich hohe Acidität aus. Die Synthese dieser Zeolithe erfolgt in bekannter Weise aus einer Silicium- und einer Aluminium- bzw. Bor-Komponente in Gegenwart von voluminösen quaternären organischen Ammonium- und Alkaliverbindungen als Mineralisierungsmittel. Ein derartiges Verfahren ist in US-PS-3 702 886 beschrieben. Die großen Ammoniumionen sollen als Templat für die gewünschte Zeolithstruktur wirken und ermöglichen die Synthese von Aluminium-Zeolithen mit $SiO_2/Al_2O_3$-Verhältnissen z. B. von 10 bis 3 000 und von Bor-Zeolithen mit $SiO_2/B_2O$-Verhältnissen z. B. von 10 bis 250.

Gegenstand der Erfindung sind neue Niob-haltige Zeolithe vom Pentasiltyp.

Im besonderen sind Gegenstand der Erfindung Niob-haltige Alumino- und Borosilikatzeolithe der allgemeinen Formel

$$M_{2/n}O : W_2O_3 : x\ Nb_2O_5 : y\ SiO_2 : z\ H_2O,$$

worin M zumindest ein Kation aus der Gruppe H, Alkali und Erdalkali mit einer Wertigkeit n = 1 oder 2 und W = Al oder B bedeutet, x = 0,5 bis 20 y $\geqslant$ 10 betragen und z zwischen 0 und 160 liegt.

Kennzeichnend für die erfindungsgemäßen neuartigen Niob-haltigen Zeolithe vom Pentasiltyp sind folgende vereinfachte Röntgenbeugungsdiagramme :

a) Niob-Aluminosilikatzeolith

| Interplanarer Netzebenenabstand d(Å) | Relative Intensität $I/I^o$ |
|---|---|
| 11.0932 | 100 |
| 9.9649 | 82 |
| 6.6789 | 10 |
| 5.9677 | 19 |
| 3.8380 | 89 |
| 3.7387 | 30 |
| 3.7092 | 42 |
| 3.6426 | 19 |
| 3.5967 | 8 |

b) Niob-Borosilikatzeolithe

| Interplanare Netzebenenabstände d(Å) | Relative Intensität $I/I^0$ |
|---|---|
| 11.0584 | 91 |
| 9.9808 | 70 |
| 9.6738 | 22 |
| 6.3181 | 16 |
| 5.9548 | 24 |
| 5.6727 | 12 |
| 5.5379 | 17 |
| 3.8314 | 100 |
| 3.7985 | 70 |
| 3.7270 | 31 |
| 3.6986 | 46 |
| 3.6260 | 28 |

Es wurde gefunden, daß man diese neuartigen Niob-haltigen Alumino- bzw. Borosilikatzeolithe vom Pentasiltyp durch hydrothermale Kristallisation erhält, wenn man die Synthese mit $SiO_2$, $Al(OH)_3$ und/oder $H_3BO_3$ und $Nb_2O_5$ bei Temperaturen von 80 °C bis 200 °C in etherischer oder alkoholischer oder aminhaltiger Lösung ausführt.

Die Synthese kann in Gegenwart von Alkali und/oder Erdalkali ausgeführt werden, aber auch der alkalifreie Weg direkt zur H-Form der Zeolithe ist möglich, wie die nachfolgenden Beispiele zeigen.

Im ersteren Falle kann M laut obiger Formel z. B. Na, K, Ca oder Mg bedeuten, im letzteren Falle steht H für M.

Besonders geeignete Lösungs- bzw. Kristallisationsmittel sind Alkohole, Ether und Amine. Als Lösungsmittel kann man allgemein ein-, zwei- oder mehrwertige, primäre, sekundäre oder tertiäre Alkohole, z. B. $CH_3OH$, $CH_3CH_2OH$, $(CH_3)_2CH—OH$, Butandiol, Hexandiol einsetzen, vorzugsweise verwendet man 1,4-Butandiol und/oder 1,6-Hexandiol.

Als etherische Lösungsmittel kann man sowohl lineare als auch cyclische Ether mit einer $(—CH_2—CH_2—O)$-Gruppierung wie Mono-, Di-, Tri- und Tetraethylenglykoldimethylether (Glyme), Diethylether, Tetrahydrofuran, Dioxan oder ein Gemisch von Tri- bis Dekaethylenglykol-methylisopropylethern des mittleren Ethoxylierungsgrades 5,5 der Formel $CH_3O—(CH_2CH_2—O)_{5,5}—C_3H_7$, Diethylenglykolmethyl-isopropylether oder ein Gemisch von Polyethylenglykolmethyl-isopropylethern des mittleren Ethoxylierungsgrades 21 oder ein Gemisch von Tri- bis Dekaethylenglykol-dimethylethern des mittleren Ethoxylierungsgrades 5,5 der Formel $CH_3O(—CH_2—CH_2—O)_{5,5}$ oder ein Gemisch von ethoxylierten Oxoalkoholen mit mittlerem Molekulargewicht von 200, 600, 6 000 sowie lineare mit einer $(CH_3—CH—CH_2—O)$-Gruppierung sowie lineare mit einer $(—CH_2—O)$-Gruppierung wie Dimethoxymethan. Bei Verwendung von Ethergemischen wie Sepasolv, Selexol und den Plurioltypen, ist die Herstellung schwieriger als bei Verwendung von Ethern mit definierter Kettenlänge. Insbesondere ist Diethylenglykoldimethylether geeignet.

Als Amine kommen zum Einsatz primäre oder sekundäre Amine wie Dipropylentriamin, Dihexamethylentriamin, Hexamethylendiamin, Propylendiamin, Diethylentriamin, Triethylentriamin oder Mischungen dieser Amine.

Eine bevorzugte Ausführungsform zur Herstellung des Niob-Aluminosilikatzeolithen besteht darin, daß man eine Reaktionsmischung aus $SiO_2$, z. B. eine pyrogene Kieselsäure, frisch gefälltes $Al(OH)_3$ und Niob(V)-oxid in einer Amin/Wasser-Mischung von 50 : 50 Gew.% 5 bis 7 Tage bei 130 °C bis 170 °C unter autogenem Druck in einem Rührautoklaven umsetzt. Das zunächst erhaltene Reaktionsprodukt liegt vor der Calcination in der Ammonium-Form vor und kann anstelle der in den intrakristallinen Poren vorhandenen Wasser-Molekeln noch Amine enthalten. Alle Reste organischer Verbindungen werden durch Trocknen und Calcinieren entfernt, so daß die H-Form des Zeolithen erhalten wird. Die abgetrennte Mutterlauge kann man für weitere Synthesen verwenden.

Die bevorzugte Ausführungsform zur Herstellung des Niob-Borosilikatzeolithen besteht darin, daß man eine Reaktionsmischung aus $SiO_2$, z. B. käufliche pyrogene Kieselsäure, Borsäure, Niob-V-oxid und Natronlauge in einer Ether/Wasser-Mischung oder einer Alkohol/Wasser-Mischung von 50 : 50 Gew.% 5 bis 7 Tage bei 110 bis 130 °C unter autogenem Druck in einem Rührautoklaven umsetzt. Das zunächst erhaltene Reaktionsprodukt kann vor dem Calcinieren noch Ether bzw. Alkohol enthalten anstelle der in

den intrakristallinen Poren enthaltenen Wasser-Molekel. Alle Reste organischer Verbindungen werden durch Trocknen und Calcinieren entfernt, so daß nur noch die freien Alkaliionen im Zeolith vorhanden sind. Die Überführung der Alkali-Form des Zeolithen in die katalytisch aktive H-Form kann nach bekannten Austauschverfahren, z. B. mit Ammoniumsalzen erfolgen. Es ist vorteilhaft, daß die abgetrennte Mutterlauge in vollem Umfang wieder zur Synthese neuer Zeolithe eingesetzt werden kann.

Die erfindungsgemäßen neuartigen kristallinen Niob-haltigen Zeolithe vom Pentasiltyp sind besonders geeignet als Katalysatoren für Kohlenwasserstoffumwandlungen für die Herstellung von Olefinen und/oder Aromaten aus Methanol und/oder Dimethylether und für die Alkylierung von Aromaten.

Die erfindungsgemäß hergestellten Zeolithe zeigen die in der nach Tabelle 1 und 2 aufgeführten vollständigen Röntgenbeugungslinien.

Das Röntgenbeugungsdiagramm wird mit einem automatischen Pulverdiffraktometer APD-10 hergestellt. Es wird Kupferstrahlung zusammen mit einem Graphitmonochromator verwendet.

Tabelle 1

Niob-Aluminiumsilikatzeolith

| Interplanare Netzebenenabstände d(Å) | relative Intensität $I/I^{o}$ |
|---|---|
| 11.0832 | 100 |
| 9.9649 | 82 |
| 7.3887 | 5 |
| 6.6789 | 10 |
| 6.3505 | 9 |
| 6.1057 | 6 |
| 5.9677 | 19 |
| 5.6866 | 9 |
| 5.5529 | 14 |
| 4.9842 | 10 |
| 4.6037 | 7 |
| 4.3491 | 10 |
| 4.2585 | 6 |
| 3.9950 | 4 |
| 3.8380 | 89 |
| 3.7387 | 30 |
| 3.7092 | 42 |
| 3.6426 | 19 |
| 3.5967 | 8 |
| 3.4788 | 6 |
| 3.3490 | 8 |
| 3.3087 | 9 |
| 3.0447 | 9 |
| 2.9793 | 12 |
| 2.9393 | 4 |
| 2.8581 | 3 |
| 2.7771 | 3 |
| 2.7591 | 3 |
| 2.6074 | 4 |

Tabelle 1 (Fortsetzung)

| Interplanare Netzebenenabstände d(Å) | relative Intensität $I/I^{o}$ |
|---|---|
| 2.5091 | 3 |
| 2.4858 | 5 |
| 2.4149 | 4 |
| 2.4055 | 4 |
| 2.3955 | 4 |
| 2.0768 | 2 |
| 2.0559 | 2 |
| 2.0398 | 1 |
| 2.0076 | 9 |
| 1.9928 | 8 |
| 1.9509 | 2 |
| 1.9125 | 3 |
| 1.8706 | 4 |
| 1.7658 | 2 |
| 1.7592 | 2 |
| 1.7028 | 2 |
| 1.6871 | 2 |
| 1.6738 | 2 |
| 1.6676 | 3 |
| 1.6592 | 3 |
| 1.6553 | 2 |
| 1.5625 | 1 |
| 1.4881 | 2 |
| 1.4594 | 0 |
| 1.4521 | 2 |

Tabelle 2

Niob-Borosilikatzeolith

| Interplanare Netzebenenabstände d(Å) | relative Intensität $I/I^{o}$ |
|---|---|
| 11.0584 | 91 |
| 9.9808 | 70 |
| 9.6738 | 22 |
| 6.6594 | 10 |
| 6.3181 | 16 |
| 5.9548 | 24 |
| 5.6727 | 12 |
| 5.5379 | 17 |

Tabelle 2 (Fortsetzung)

| Interplanare Netzebenenabstände d(Å) | relative Intensität $I/I^o$ |
|---|---|
| 4.9897 | 10 |
| 4.5885 | 8 |
| 4.3354 | 11 |
| 5.2315 | 12 |
| 3.9832 | 6 |
| 3.8314 | 100 |
| 3.7985 | 70 |
| 3.7270 | 31 |
| 3.6986 | 46 |
| 3.6260 | 28 |
| 3.4640 | 6 |
| 3.4205 | 8 |
| 3.3308 | 10 |
| 3.2956 | 10 |
| 3.2409 | 4 |
| 3.1571 | 3 |
| 3.0342 | 12 |
| 2.9735 | 11 |
| 2.8406 | 3 |
| 2.7815 | 4 |
| 2.7160 | 4 |
| 2.5938 | 6 |
| 2.5726 | 3 |
| 2.5557 | 2 |
| 2.5012 | 3 |
| 2.4757 | 5 |
| 2.4020 | 4 |
| 2.3841 | 4 |
| 2.3192 | 3 |
| 2.0782 | 2 |
| 2.0572 | 2 |
| 2.0010 | 10 |
| 1.9803 | 9 |
| 1.9405 | 3 |
| 1.9108 | 3 |
| 1.9042 | 3 |
| 1.8630 | 3 |
| 1.7571 | 2 |
| 1.6603 | 0 |

Tabelle 2 (Fortsetzung)

| Interplanare Netzebenenabstände d(Å) | relative Intensität $I/I^o$ |
|---|---|
| 1.6554 | 2 |
| 1.5567 | 2 |
| 1.4544 | 4 |
| 1.4454 | 2 |
| 1.4378 | 3 |
| 1.4152 | 2 |

Die für die erfindungsgemäß hergestellten Niob-Alumino- und Niob-Borosilikatzeolithe typischen Röntgenbeugungsmuster weisen diese als Mitglieder der Pentasil-Familie aus.

Die in der H-Form vorliegenden erfindungsgemäßen Zeolithe können mit Matrixmaterial verstrangt und als Katalysator für Kohlenwasserstoffumwandlungen wie Isomerisierung, Cracken und Umwandlung von Alkoholen und Ethern eingesetzt werden.

Die Durchführung des erfindungsgemäßen Verfahrens wird anhand der nachstehenden Beispiele näher erläutert.

Die in den Beispielen angegebenen Analysenwerte beziehen sich auf Trockenbasis. Die Substanzen werden vor der chemischen Analyse bei 550 °C/20 h calciniert. Die Differenz zu 100 % ergibt sich durch absorbiertes Wasser.

Beispiel 1

In 800 g 50 %iger Hexamethylendiamin-Lösung (HMD) werden bei 60 °C 80 g Aerosil eingerührt. Aus 25 g $Al(NO_3)_3 \cdot 9\,H_2O$ läßt sich mit Ammoniak $Al(OH)_3$ fällen. Dieses frisch gefällte Aluminiumhydroxid wird abfiltriert, gut mit Wasser ausgewaschen und in 200 g 50 %iger HMD-Lösung aufgenommen. Beide Lösungen werden zusammengegeben. Dem Gemisch werden 2 g Niob-V-oxid zugesetzt. Die homogene gerührte Mischung wird in einem Rührautoklaven 5 Tage bei 150 bis 170 °C unter autogenem Druck umgesetzt. Nach Abkühlen des Reaktionsgemisches wird das kristalline Produkt abfiltriert, mit 10 l $H_2O$ ausgewaschen, 16 h bei 110 °C getrocknet und bei 20 h bei 500 °C calciniert.

Der erhaltene Zeolith setzt sich zusammen aus 88,6 Gew.% $SiO_2$, 3,2 Gew.% $Al_2O_3$ und 1,4 Gew.% Nb.

Das Röntgenbeugungsdiagramm des calcinierten Katalysators ist in Tabelle 1 wiedergegeben.

Beispiel 2

In 1 000 g Diglyme/Wasser-Gemisch (50 : 50 Gew.%) werden bei ca. 40 °C 80 g Aerosil eingerührt. 15,2 g $H_3BO_3$ werden in 200 g Diglyme/Wasser-Gemisch gelöst. Beide Lösungen werden zusammengegeben. Danach werden 14,7 g NaOH, 2 g $Nb_2O_5$ und eventuell Impfkristalle zugesetzt. Die homogene gerührte Mischung wird in einem Rührautoklaven 5 Tage bei Temperaturen zwischen 80 °C und 140 °C unter autogenem Druck umgesetzt. Nach Abkühlen des Reaktionsgemisches wird das kristalline Produkt abfiltriert, mit 10 l Wasser ausgewaschen, 16 h bei 110 °C getrocknet und 20 h bei 500 °C calciniert.

Der erhaltene Zeolith setzt sich zusammen aus 79,7 Gew.% $SiO_2$, 6,55 Gew.% $B_2O_3$, 1,4 Gew.% Nb und 7,4 Gew.% $Na_2O$.

Das Röntgenbeugungsdiagramm des calcinierten Katalysators entspricht dem in Tabelle 2.

Beispiel 3

Der in Beispiel 2 hergestellte Niob-Borosilikatzeolith wird mit Böhmit im Verhältnis 60 : 40 verstrangt. Das Überführen der Na-Form in die katalytisch aktive H-Form des Zeolithen erfolgt durch Austausch mit $NH_4Cl$ (20 %iger $NH_4Cl$-Lösung) im Verhältnis 1 : 15 in 2 h bei 80 °C.

An diesem Katalysator wird bei 500 °C und einer Belastung WHSV = 7,8 $h^{-1}$ 80 %iges Methanol quantitativ umgesetzt.

Die Produktausbeuten, bezogen auf eingesetztes $CH_2$ sind

| | | | |
|---|---|---|---|
| $CH_4$ | 4,2 % | $C_2H_4$ | 11,4 % |
| $C_2H_6$ | 0,4 % | $C_3H_6$ | 34,5 % |
| $C_3H_8$ | 2,2 % | $C_4$-KW | 13,1 % |
| $C_5^+$-KW | 32,0 % | | |

**Patentansprüche**

1. Niob-haltige Zeolithe vom Pentasiltyp.
2. Niob-haltige Alumino- und Borosilikatzeolithe nach Anspruch 1 der allgemeinen Formel

$$M_{2/n}O : W_2O_3 : x\ Nb_2O_5 : y\ SiO_2 : z\ H_2O\ ,$$

worin M zumindest ein Kation aus der Gruppe H, Alkali und Erdalkali mit einer Wertigkeit n = 1 oder 2 und W = Al und/oder B bedeutet, x = 0,5 bis 20 und y ≥ 10 betragen und z zwischen 0 und 160 liegt.

3. Verfahren zur Herstellung von Niob-haltigen kristallinen Alumino- und Borosilikatzeolithen gemäß Anspruch 1 und 2 durch hydrothermale Kristallisation, dadurch gekennzeichnet, daß man die Synthese aus $SiO_2$, $Al(OH)_3$ und/oder $H_3BO_3$ und $Nb_2O_5$ bei Temperaturen von 80 °C bis 200 °C in etherischer oder alkoholischer oder aminhaltiger Lösung ausführt.
4. Verfahren nach Anspruch 3, dadurch gekennzeichnet, daß man als Lösungsmittel für die Kristallisation Diethylenglykoldimethylether verwendet.
5. Verfahren nach Anspruch 3, dadurch gekennzeichnet, daß man als Lösungsmittel für die Kristallisation 1,4-Butandiol und/oder 1,6-Hexandiol verwendet.
6. Verfahren nach Anspruch 3, dadurch gekennzeichnet, daß man als Lösungsmittel für die Kristallisation Hexamethylendiamin verwendet.
7. Verwendung von kristallinen Niob-Alumino- bzw. Niob-Borosilikatzeolithe gemäß Anspruch 1 bis 6 für Kohlenwasserstoffumwandlungen.
8. Verwendung von kristallinen Niob-Alumino- bzw. Niob-Borosilikatzeolithen gemäß Anspruch 7 für die Herstellung von Olefinen aus Methanol und/oder Dimethylether.
9. Verwendung von kristallinen Niob-Alumino- bzw. Niob-Borosilikatzeolithen gemäß Anspruch 7 für die Alkylierung von Aromaten.

**Claims**

1. Niobium-containing zeolites of the pentasil type.
2. Niobium-containing aluminosilicate and borosilicate zeolites as claimed in claim 1, of the general formula

$$M_{2/n}O : W_2O_3 : x\ Nb_2O_5 : y\ SiO_2 : z\ H_2O\ ,$$

where M is at least one cation selected from the group consisting of H, alkali metal and alkaline earth metal having a valency n of 1 or 2, and W is Al and/or B, x is 0.5 to 20, y ≥ 10, and z is between 0 and 160.

3. A process for the production of niobium-containing crystalline aluminosilicate and borosilicate zeolites as claimed in claims 1 and 2 by hydrothermal crystallization, wherein the synthesis from $SiO_2$, $Al(OH)_3$ and/or $H_3BO_3$ and $Nb_2O_5$ is carried out at a temperature of from 80 to 200 °C in an ethereal, alcoholic or amine-containing solution.
4. A process as claimed in claim 3, wherein diethylene glycol dimethyl ether is used as solvent for the crystallization.
5. A process as claimed in claim 3, wherein 1,4-butanediol and/or 1,6-hexanediol is used as solvent for the crystallization.
6. A process as claimed in claim 3, wherein hexamethylenediamine is used as solvent for the crystallization.
7. The use of crystalline niobium-containing aluminosilicate and borosilicate zeolites as claimed in claim 1 to 6 for hydrocarbon conversions.
8. The use of crystalline niobium-containing aluminosilicate and borosilicate zeolites, as claimed in claim 7, for the production of olefins from methanol and/or dimethyl ether.
9. The use of crystalline niobium-containing aluminosilicate and borosilicate zeolites, as claimed in claim 7, for the alkylation of aromatics.

**Revendications**

1. Zéolithes au niobium du type pentasil.
2. Zéolithes d'alumino- et de boro-silicates au niobium selon la revendication 1, de la formule générale

$$M_{2/n}O : W_2O_3 : x\ Nb_2O_5 : y\ SiO_2 : z\ H_2O$$

dans laquelle M représente au moins un cation hydrogène, métal alcalin ou métal alcalino-terreux avec une valence n égale à 1 ou 2, W = Al et (ou) B, x = 0,5 à 20, y ≥ 10 et z vaut entre 0 et 160.

3. Procédé de préparation de zéolithes d'alumino- et de boro-silicates au niobium cristallisées selon la revendication 1 et la revendication 2 par cristallisation hydrothermique, caractérisé en ce que la synthèse au départ de $SiO_2$, $Al(OH)_3$ et (ou) $H_3BO_3$ et de $Nb_2O_5$ est réalisée en solution éthérée ou alcoolique ou en solution d'amine à des températures de 80 à 200 °C.

4. Procédé selon la revendication 3, caractérisé en ce que le solvant pour la cristallisation est l'éther diméthylique du diéthylène-glycol.

5. Procédé suivant la revendication 3, caractérisé en ce que le solvant pour la cristallisation est le butane-diol-1,4 et(ou) l'hexane-diol-1,6.

6. Procédé suivant la revendication 3, caractérisé en ce que le solvant pour la cristallisation est l'hexaméthylène-diamine.

7. Utilisation de zéolithes d'alumino- et de boro-silicates au niobium cristallisées selon les revendications 1 à 6 pour des réactions de transformation d'hydrocarbures.

8. Utilisation de zéolithes d'alumino- et de boro-silicates au niobium cristallisées selon la revendication 7 pour la préparation d'oléfines à partir de l'alcool méthylique et(ou) de l'éther diméthylique.

9. Utilisation de zéolithes d'alumino- et de boro-silicates au niobium cristallisées selon la revendication 7 pour l'alkylation de composés aromatiques.